(19) Europäisches Patentamt
European Patent Office
Office européen des brevets

(11) Veröffentlichungsnummer: **0 229 642 B1**

(12) # EUROPÄISCHE PATENTSCHRIFT

(45) Veröffentlichungstag der Patentschrift: **11.07.90**

(51) Int. Cl.[5]: **C07D 249/08**, C07D 233/60, C07D 233/61, C07D 405/06, A01N 43/653, A01N 43/50

(21) Anmeldenummer: **87100183.0**

(22) Anmeldetag: **09.01.87**

(54) **Azolverbindungen und diese enthaltende Fungizide und Wachstumsregulatoren.**

(30) Priorität: **14.01.86 DE 3600812**

(43) Veröffentlichungstag der Anmeldung: **22.07.87 Patentblatt 87/30**

(45) Bekanntmachung des Hinweises auf die Patenterteilung: **11.07.90 Patentblatt 90/28**

(84) Benannte Vertragsstaaten: **AT BE DE ES FR GB GR IT NL SE**

(56) Entgegenhaltungen:
**EP-A- 0 103 798**
**EP-A- 0 135 083**
**EP-A- 0 203 440**
**DE-A- 2 805 227**
**DE-A- 3 025 879**
**DE-A- 3 220 183**

(73) Patentinhaber: **BASF Aktiengesellschaft, Carl-Bosch-Strasse 38, D-6700 Ludwigshafen(DE)**

(72) Erfinder: **Rentzea, Costin, Dr., Richard-Kuhn-Strasse 1-3, D-6900 Heidelberg(DE)**
Erfinder: **Schulz, Günter, Dr., Carl-Bosch-Strasse 96, D-6700 Ludwigshafen(DE)**
Erfinder: **Tuerk, Wolfgang, Dr., Wittelsbachstrasse 61, D-6700 Ludwigshafen(DE)**
Erfinder: **Ammermann, Eberhard, Dr., Sachsenstrasse 3,, D-6700 Ludwigshafen(DE)**
Erfinder: **Pommer, Ernst-Heinrich, Dr., Berliner Platz 7, D-6703 Limburgerhof(DE)**
Erfinder: **Rademacher, Wilhelm, Dr., Austrasse 1, D-6703 Limburgerhof(DE)**
Erfinder: **Jung, Johann, Prof.Dr., Hardenburgstrasse 19, D-6703 Limburgerhof(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 229 642 B1

ACTORUM AG

**Beschreibung**

Die vorliegende Erfindung betrifft neue Azolverbindungen, Verfahren zu ihrer Herstellung und die Verwendung der Wirkstoffe zur Bekämpfung von Pilzen und zur Regulierung des Pflanzenwachstums.

Es ist bekannt, daß 2,2-Dimethyl-4-(1H-1,2,4-triazol-1-yl)-6-hepten-3-on fungizide Eigenschaften besitzt (DE-OS 2 638 470 und DE-OS 2 805 227).

Desweiteren sind aus der EP-A 0 103 798 Azolylalkyl-tert.-butylketone und -carbinole als Fungizide und als Zwischenprodukte für die Synthese weiterer Pflanzenschutzmittel bekannt.

Zusätzlich sind aus der EP-A 0 135 083 fungizide 3-Cycloalkyl-1-(1,3-dioxan-5-yl)-2-(1,2,4-triazol-1-yl)-propan-1-one und -propan-1-ole und aus der EP-A2 0 203 440 fungizide Dichlorcyclopropylalkyl-hydroxyalkyl-azol-Derivate bekannt.

Die fungizide Wirkung dieser Verbindungen ist jedoch bei manchen Pflanzenarten und bei niedrigen Aufwandmengen nicht immer voll befriedigend.

Es wurden nun neue Verbindungen gefunden, die eine hohe fungizide Wirkung bei guter Pflanzenverträglichkeit aufweisen, die besser ist als die Wirkung der bekannten Verbindung, und zwar Verbindungen der Formel I

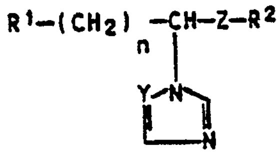

I,

worin

$R^1$ eine Gruppe der Formel

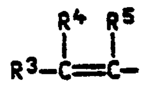

mit cis- oder trans-Doppelbindung bedeutet, wobei

$R^3$, $R^4$ und $R^5$ gleich oder verschieden sind und Wasserstoff, Fluor, Chlor, Brom, Methyl oder Ethyl bedeuten,

n eine ganze Zahl von 1 bis 3 bedeutet,

Y ≥CH oder ≥N ist,

$R^2$ tert.-Butyl, 5-Methyl-1,3-dioxan-5-yl, ggf. durch Methyl substituiertes 1-Methylcyclohexyl und ggf. durch Methyl substituiertes 2-Methyltetrahydropyran-2-yl oder die Gruppe

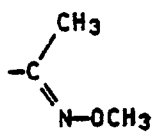

bedeutet, und

Z die Gruppe >C=O, >C=N–$OR^8$ oder >$CR^6$–$OR^7$ bedeutet, mit der Maßgabe, daß nicht gleichzeitig $R^1$ $CH_2$=CH–, n = 1, Z >C=O und $R^2$ tert.-Butyl ist, und

$R^6$ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und

$R^7$ Wasserstoff, $C_2$–$C_4$-Alkanoyl oder Benzoyl, $R^8$ Wasserstoff oder Methyl bedeutet, sowie deren für Pflanzen verträgliche Säureadditionssalze und Metall-Komplexe.

In den Verbindungen der Formel I sind das azolylsubstituierte Kohlenstoffatom und das benachbarte durch $R^6$ und $OR^7$ substituierte Kohlenstoffatom chiral, die Doppelbindung kann cis- oder transfiguriert sein; die Wirkstoffe fallen demgemäß grundsätzlich als Enantiomerengemische an, die in die optisch aktiven Enantiomere getrennt werden können. Bedingt durch die Anwesenheit zweier chiraler Zentren fallen die Wirkstoffe auch als Diastereomerengemische an, die in üblicher Weise, z.B. durch Chromatographie oder Kristallisation in die einzelnen, einheitlichen Diastereomeren getrennt werden können. Für die Anwendung der Verbindungen der Formel I als Fungizide oder als Pflanzenwachstumsregulatoren ist jedoch eine Trennung der Enantiomeren oder Diastereomeren normalerweise nicht erforderlich. Die Erfindung umfaßt sowohl die optisch aktiven einheitlichen Substanzen als auch deren Gemische.

Geeignete Säureadditionssalze sind beispielsweise die Chloride, Bromide, Sulfate, Methylsulfonate, Nitrate, Phosphate, Oxalate oder Dodecylbenzolsulfonate. Die Wirksamkeit der Salze geht auf das Kation zurück, so daß die Wahl des Anions beliebig ist.

Die Azolylverbindungen der Formel I gemäß Anspruch 1, mit der Maßgabe, daß Z >$CR^6$–$OR^7$ und $R^6$ Wasserstoff ist, werden vorzugsweise durch stereoselektive Reduktion der Ketone der Formel II und anschließende Acylierung gemäß Anspruch 2 dargestellt.

Dabei entstehen bei der Reduktion mit komplexen Hydriden, vorzugsweise mit Natriumborhydrid in einem protischen Verdünnungsmittel, vorzugsweise Methanol, Ethanol oder i-Propanol, stark überwiegend die R*,R*-diastereomeren Alkohole der Formel I wobei in der Gruppe Z = $CR^6OR^7$, $R^6$ und $R^7$ Wasserstoff sind. Man erhält vorwiegend die diastereomeren Triazolylalkohole der R*,S*-Konfiguration, wenn man die Ketone der Formel II reduziert

a) mit sekundären Alkoholaten oder
b) mit Alkylmagnesiumhalogeniden oder
c) mit Wasserstoff in Gegenwart geeigneter Hydrierkatalysatoren.

Zur Erläuterung sei vermerkt, daß die Verbindungen mit zwei verschiedenen chiralen Kohlenstoffatomen vier Stereoisomere bilden, deren Konfiguration nach dem Cahn-Ingold-Prelog-System bezeichnet werden kann (siehe z.B. D. Seebach und V. Prelog, Angew. Chem. 94, 696 (1982) und dort angegebene Literatur). Hierbei stellen zwei Enantiomere der Konfigurationen R,R und S,S ein Diastereomer mit der Bezeichnung R*,R* dar. Das andere Diastereomer mit der Bezeichnung R*,S* setzt sich aus den beiden Enantiomeren mit den Konfigurationen R,S und S,R zusammen.

Die erhaltenen Alkohole der Formel I mit Z = CHOH enthalten einen erheblich höheren Anteil der Diastereomeren mit R*,S*-Konfiguration als derjenigen mit R*,R*-Konfiguration. Der Anteil der R*,R*-Diastereomeren im Gemisch liegt in der Regel unter 30%. Reine R*,S*-Diastereomeren können daraus durch einfaches Waschen oder Rohprodukte mit geeigneten Lösungsmitteln, wie z.B. Diisopropylether, oder durch Umkristallisieren oder durch andere, übliche Reinigungsschritte, z.B. Chromatographie erhalten werden.

Zur stereoselektiven Reduktion der Ketone der Formel II zu den R*,S*-Diastereomeren der Formel I mit Z = CHOH geht man nach a) folgendermaßen vor:

Die Ketone der Formel II (1 Moläquivalent) werden mit sekundären Alkoholaten (vorzugsweise 0,3 bis 1,5 Moläquivalente), vorzugsweise des Aluminiums, wie z.B. Aluminiumisopropylat, Aluminiumbutylat-(2) oder Aluminiumcyclohexylat in Gegenwart eines Verdünnungsmittels bei 60 bis 160°C, vorzugsweise bei der Siedetemperatur des Verdünnungsmittels, umgesetzt. Als Verdünnungsmittel eignen sich inerte organische Lösungsmittel, insbesondere Alkohole wie Isopropanol oder Cyclohexanol. Die entstandenen diastereomeren Alkoholate werden sodann mit Hilfe von Säuren in üblicher Weise zu den freien Alkoholen der Formel I mit Z = CHOH hydrolysiert.

Für die stereoselektive Reduktion in der Ausführungsform b) des oben erwähnten Verfahrens werden die Ketone der Formel II (1 Moläquivalent) mit Alkylmagnesiumhalogeniden, die mindestens ein β-H-Atom enthalten, vorzugsweise mit 0,7 bis 1,5 Moläquivalent, in Gegenwart eines Verdünnungsmittels bei Temperaturen zwischen 0 bis 120°C, umgesetzt. Als Alkylmagnesiumhalogenide kommen z.B. Ethylmagnesiumchlorid, Isopropylmagnesiumbromid, n-Propylmagnesiumbromid oder Isobutylmagnesiumchlorid in Frage. Als Lösungsmittel für die Grignard-Reduktion kommen bevorzugt Ether in Frage, wie Diethylether, Di-n-propylether, Tetrahydrofuran oder Anisol, ferner tertiäre Amine wie N,N-Diethylanilin sowie Phosphorsäure-tris(dimethylamid). Vorzugsweise kann man die Reaktion auch im Gemisch dieser Lösungsmittel mit aliphatischen oder aromatischen Kohlenwasserstoffen wie n-Hexan oder Toluol durchführen. Die Reaktionstemperaturen lassen sich je nach Lösungsmittel zwischen 0 und 120°C variieren, bevorzugt sind Temperaturen zwischen 30 und 100°C. Die hierbei primär entstandenen Magnesiumalkoholate werden sodann durch Hydrolyse mit Wasser oder verdünnten wäßrigen Säuren, wie Salzsäure, Schwefelsäure oder bevorzugt Essigsäure oder besonders bevorzugt mit wäßriger Ammoniumchloridlösung in die Alkohole übergeführt, und diese nach Entfernen der wäßrigen Phase, falls gewünscht, in üblicher Weise durch Extraktion, Umkristallisieren oder Chromatographie gereinigt.

Für die stereoselektive Reduktion in der Ausführungsform c) erfolgt die katalytische Hydrierung am besten an einem Edelmetallkontakt. Hierzu sind die Metalle der Platingruppe am besten geeignet. Man kann diese Metalle auf inerten Trägern niederschlagen, wobei ein Metallgehalt von 0,5 bis 10% im allgemeinen ausreichend ist. Unter Umständen kann man auch die reinen Metalle, zweckmäßig in fein verteilter Form einsetzen. Als besonders geeignet hat sich ein kolloid verteiltes Rutheniumoxidhydrat erwiesen, das durch Fällung aus einer wäßrigen Rutheniumtrichloridlösung beim pH = 8 gewonnen wurde. Die oxidische Form geht unter den Reaktionsbedingungen in den aktiven Katalysator über.

Als Lösungsmittel dienen vorzugsweise Ether wie Dioxan, Tetrahydrofuran oder Glycoldimethylether.

Die Temperatur läßt sich in weiten Grenzen verändern. Als besonders geeignet erwiesen sich Temperaturen zwischen 100 und 150°C. Bei diesen Temperaturen muß man unter Druck arbeiten; es genügen Drücke von 10 bis 30 bar. Unter höheren Drücken leidet die Selektivität.

Die Kontaktmenge ist nicht kritisch und hängt neben der Aktivität von der Reaktionstemperatur und dem Wasserstoffpartialdruck ab.

Die Acylierung der Verbindungen der Formel I mit $R^6$ = H und $R^7$ = H erfolgt wie weiter unten für die Verbindungen der Formel I mit $R^6$ verschieden von H beschrieben.

Die Azolverbindungen der Formel I gemäß Anspruch 1, mit der Maßgabe, daß Z >$CR^6$–$OR^7$ bedeutet und $R^6$ nicht Wasserstoff ist, werden durch Umsetzung der Ketone der Formel II

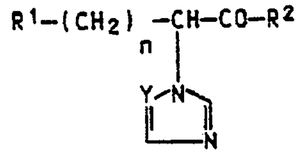

II,

mit einer Grignardverbindung der Formel III

$R^{6'}$-MgHal III,

in welcher $R^{6'}$ Methyl, Vinyl, Allyl oder Benzyl bedeutet und in der Hal Chlor, Brom oder Jod bedeutet, erhalten. Bei dieser Umsetzung verfährt man so, daß man 0,8 bis 2,4 Äquivalente der Grignardverbindung einsetzt, vorzugsweise in Anwesenheit eines Lösungs- oder Verdünnungsmittels und gegebenenfalls in Anwesenheit eines ausbeutesteigernden Salzes.

Als Lösungsmittel kommen bevorzugt Ether in Frage, wie Diethylether, Di-n-propylether, Tetrahydrofurane oder Anisol, ferner tertiäre Amine wie N,N-Diethylanilin sowie Phosphorsäure-tris(dimethylamid); gegebenenfalls kann man die Reaktion auch im Gemisch dieser Lösungsmittel mit aliphatischen oder aromatischen Kohlenwasserstoffen wie n-Hexan oder Toluol durchführen. Als ausbeutesteigernde Salze, die die üblichen Nebenreaktionen unterdrücken, kommen insbesondere wasserfreie Magnesiumhalogenide wie wasserfreie Magnesiumbromide oder wasserfreie Tetraalkylammoniumhalogenide wie z.B. Tetra-n-butylammoniumchlorid in Frage.

Die Reaktionstemperaturen lassen sich je nach Lösungsmittel zwischen 0 und 100°C variieren, bevorzugt sind Temperaturen zwischen 0 und 60°C. Die hierbei primär entstandenen Magnesiumalkoholate werden sodann durch Hydrolyse mit Wasser oder verdünnten wäßrigen Säuren, wie Salzsäure, Schwefelsäure oder bevorzugt Essigsäure oder besonders bevorzugt mit wäßriger Ammoniumchloridlösung in die Alkohole übergeführt, und diese nach Entfernen der wäßrigen Phase, falls gewünscht, in üblicher Weise durch Extraktion, Umkristallisieren oder Chromatographie gereinigt.

Das Verfahren zur Herstellung der Ester der Formel I ($R^7$ = $C_2$–$C_4$-Alkanoyl) besteht darin, daß man die sekundären oder tertiären Alkohole der Formel I (Z = $CR^6OR^7$ mit $R^7$ = H) mit den entsprechenden Säurechloriden oder Säureanhydriden in Gegenwart eines säurebindenden Mittels und gegebenenfalls in Gegenwart eines aprotischen Lösungsmittels oder Verdünnungsmittels sowie bevorzugt in Gegenwart eines Alkylierungskatalysators bei Temperaturen zwischen 0 und 100°C, vorzugsweise zwischen 10 und 50°C umsetzt. Als säurebindende Mittel können anorganische Basen wie Natriumamid oder besonders bevorzugt Pyridin in mindestens äquivalenten Mengen eingesetzt werden. Als Acylierungskatalysatoren verwendet man zweckmäßigerweise Imidazol oder 4-Dimethylaminopyridin in Mengenanteilen von 0,01 bis 0,4 Äquivalenten, falls nicht bereits Pyridin anwesend ist. Als Lösungsmittel können Kohlenwasserstoffe, wie Cyclohexan oder Toluol, Ether wie Diethylether oder auch überschüssige säurebindende Amine wie Triethylamin oder Pyridin eingesetzt werden.

Die Ketone der Formel II lassen sich herstellen, indem man bekannte Ketone der Formel IV (DE-OS 2 638 470, 3 025 879, 3 026 140 und 3 220 183) oder deren Alkalienolate mit Alkenylhalogeniden der Formel V, gegebenenfalls in Gegenwart einer Base und gegebenenfalls eines Lösungs- oder Verdünnungsmittels alkyliert.

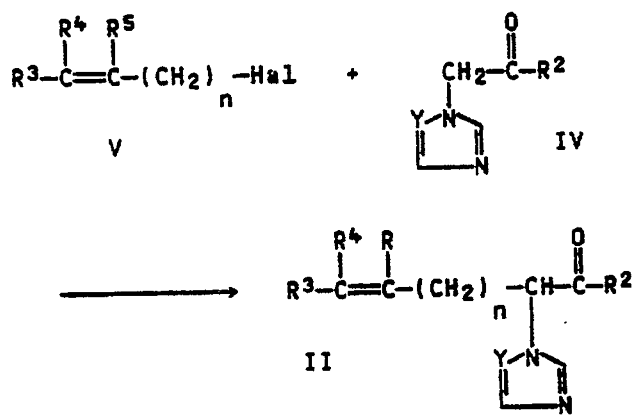

V IV II

Hierzu können die Ketone IV zunächst zu den Alkalienolaten metallisiert werden, indem man sie vorzugsweise in Gegenwart eines polaren aprotischen Lösungsmittels wie Dimethylformamid, Acetonitril oder Tetrahydrofuran mit 0,8 bis 1,2 Äquivalenten bevorzugt 1,0 Äquivalenten, eines Metallierungsreagens wie Natriumhydrid, Lithiumdiisopropylamid oder n-Butyllithium bei 0 bis 100°C, vorzugsweise bei 10

bis 50°C umsetzt. Nach anschließender Zugabe von 0,8 bis 2,0, bevorzugt 1,0 Äquivalenten des jeweiligen Alkenylhalogenides der Formel V erhält man bei Reaktionstemperaturen zwischen 0 und 100°C, bevorzugt bei 5 bis 30°C die Ketone der Formel II.

Eine Variante dieses Verfahrens besteht darin, daß man die Ketone IV in Gegenwart von 0,8 bis 1,2, bevorzugt 1,0 Äquivalenten einer Base, z.B. Kalium-tert.-butoxid, Natriummethoxid oder Kaliumhydroxid mit den Alkenylhalogeniden V umsetzt, wobei man zweckmäßigerweise in Gegenwart eines Lösungs- oder Verdünnungsmittels bei Temperaturen zwischen 0 und 100°C, bevorzugt bei 5 bis 50°C, arbeitet.

Falls gewünscht, können die neuen Verbindungen der Formel I auch in Salze mit anorganischen oder organischen Säuren übergeführt werden, wie beispielsweise in Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions beliebig ist.

Ferner lassen sich die Verbindungen der Formel I nach bekannten Methoden in Metallkomplexe überführen. Das kann durch Umsetzung dieser Verbindungen mit Metallsalzen, z.B. der Metalle Kupfer, Zink, Eisen, Mangan oder Nickel, beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Herstellungsbeispiele

Beispiel 1

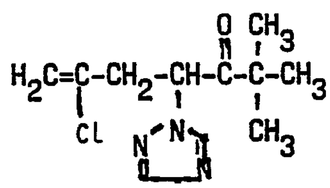

Zu einer unter Stickstoff gehaltenen Suspension von 5,3 g (0,22 mol) Natriumhydrid in 50 ml absolutem Dimethylformamid wird unter Rühren die Lösung von 33,4 g (0,2 mol) 2,2-Dimethyl-4-(1,2,4-triazolyl-(1))-butan-3-on in 50 ml absolutem Dimethylformamid bei 20 bis 25°C zugetropft. Nach dreistündigem Nachrühren wird bei 60°C die Lösung von 36 g (0,2 mol) 2,3-Dichlorprop-1-en zugetropft. Anschließend wird das Reaktionsgemisch weitere 5 Stunden unter Rückfluß erhitzt. Bei Raumtemperatur werden dann etwa 100 ml Eiswasser vorsichtig zugetropft. Es wird dreimal mit je 300 ml Methylenchlorid extrahiert. Die organische Phase wird anschließend mehrmals mit Wasser gewaschen, über $Na_2SO_4$ getrocknet und eingeengt. Man erhält 32,2 g (66,7% d. Th.) 2,2-Dimethyl-6-chlor-4-(1,2,4-triazolyl-(1))-6-hepten-3-on als weiße Kristalle vom Schmp. 78 bis 80°C (Verbindung Nr. 1).

Als Erkennungsmerkmal für die R*,R*-Diastereomeren kann z.B. dienen, daß im 1-H-NMR-Spektrum das Signal für die Protonen der tert.-Butylgruppe bei 0,5 bis 0,7 ppm auftritt, während es beim R*,S*-Diastereomeren bei 0,8 bis 1,0 ppm liegt.

Unter entsprechender Abwandlung der vorstehenden Angaben wurden die in der folgenden Tabelle aufgeführten Verbindungen hergestellt (A = R*,R*-Diastereomeres; B = R*,S*-Diastereomeres).

| Beispiel Nr. | $R^1$ | $R^2$ | Z | Diastereom. | n | Y | Fp. (°C) | IR (Film) ($cm^{-1}$) |
|---|---|---|---|---|---|---|---|---|
| 6 | $CH_2=CH-$ | tert.-$C_4H_9$- | -CHOH- | A | 1 | N | Öl | 3264, 2957, 1506, 1276, 1136, 1090, 1018, 920, 680. |
| 7 | $CH_2=CCl-$ | tert.-$C_4H_9$- | -CHOH- | A | 1 | N | Öl | 3260, 2958, 2873, 1637, 1506, 1277, 1134, 1092, 1020, 679. |
| 8 | $CH_2=CCl$ | tert.-$C_4H_9$ | -CHOH- | B | 1 | N | Öl | 3290, 2960, 1637, 1510, 1284, 1213, 1141, 1063, 1014, 885. |
| 9 | $CH_2=CBr-$ | tert.-$C_4H_9$ | -CO- | - | 1 | N | Öl | 2970, 1718, 1502, 1478, 1368, 1277, 1204, 1137, 1061, 678. |
| 10 | $CH_2=CBr$ | tert.-$C_4H_9$ | -CHOH- | A | 1 | N | Öl | 3260, 2957, 2872, 1506, 1276, 1201, 1135, 1091, 1020, 679. |
| 11 | $CH_2=CBr$ | tert.-$C_4H_9$ | -CHOH- | B | 1 | N | Öl | 3298, 2977, 2958, 1510, 1283, 1214, 1149, 1141, 1063, 1015. |
| 12 | Cl-CH=CH- | tert.-$C_4H_9$ | -CO- | - | 1 | N | Öl | 2857, 1720, 1503, 1277, 1165, 1088, 1032, 927, 679. |
| 13 | Cl-CH=CH- | tert.-$C_4H_9$ | -CHOH- | A | 1 | N | 107-109 | |
| 14 | Cl-CH=CH- | tert.-$C_4H_9$ | -CHOH- | B | 1 | N | Öl | |
| 15 | $H_3C-C(Cl)=CH-$ | tert.-$C_4H_9$ | -CO- | - | 1 | N | Öl | 2971, 1717, 1502, 1478, 1368, 1277, 1139, 1066, 1009, 678. |
| 16 | $H_3C-C(Cl)=CH-$ | tert.-$C_4H_9$ | -CHOH- | A | 1 | N | Öl | 3261, 2958, 2872, 1506, 1374, 1277, 1135, 1117, 1096, 680. |
| 17 | $H_3C-C(Cl)=CH-$ | tert.-$C_4H_9$ | -CHOH- | B | 1 | N | Öl | 3287, 3100, 2977, 2955, 1509, 1284, 1211, 1146, 1138, 1064. |

| Beispiel Nr. | R¹ | R² | Z | Diastereom. | n | Y | Fp. (°C) | IR (Film) (cm⁻¹) |
|---|---|---|---|---|---|---|---|---|
| 18 | $CH_2CH-$ | 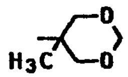 | -CO- | - | 1 | N | 62-64 | |
| 19 | $CH_2=CH$ | 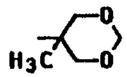 | -CHOH- | A | 1 | N | 84-86 | |
| 20 | $CH_2=CCl-$ | 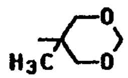 | -CO- | - | 1 | N | Öl | 2860, 1721, 1504, 1276, 1209, 1165, 1138, 1031, 926, 679. |
| 21 | $CH_2=CCl-$ | 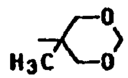 | -CHOH- | A | 1 | N | 143-146 | |
| 22 | Cl-CH=CH- | 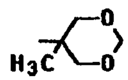 | -CO- | - | 1 | N | Öl | |
| 23 | Cl-CH=CH- | 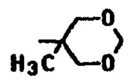 | -CHOH- | A | 1 | N | 129-132 | |
| 24 | $H_3C-C(Cl)=CH-$ | 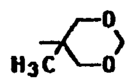 | -CO- | - | 1 | N | Öl | 2855, 1720, 1503, 1278, 1166 1140, 1088, 1034, 927, 679. |
| 25 | $H_3C-C=CH-$ | 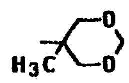 | -CHOH | A | 1 | N | 114-116 | |
| 26 | $Cl_2C=CCl-$ | 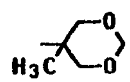 | -CO- | - | 1 | N | Öl | |
| 27 | $Cl_2C=CCl-$ | 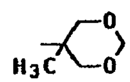 | -CHOH- | A | 1 | N | Öl | |
| 28 | $Cl_2C=CCl-$ | 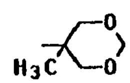 | -CHOH | B | 1 | N | Öl | |

| Beispiel Nr. | $R^1$ | $R^2$ | Z | Diastereom. | n | Y | Fp. ($^0C$) | IR (Film) ($cm^{-1}$) |
|---|---|---|---|---|---|---|---|---|
| 29 | $H_2C=CH-$ | 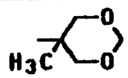 | -CO- | - | 2 | N | | |
| 30 | $H_2C=CH-$ | 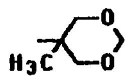 | -CHOH- | A | 2 | N | | |
| 31 | $H_2C=CH-$ | 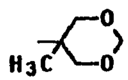 | -CHOH- | B | 2 | N | | |
| 32 | $H_2C=CH-$ | 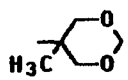 | -CO- | - | 3 | N | | |
| 33 | $H_2C=CH-$ | 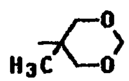 | -CHOH- | A | 3 | N | | |
| 34 | $H_2C=CH-$ | 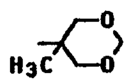 | -CHOH- | B | 3 | N | | |
| 35 | $H_2C=CH-$ | 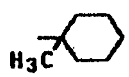 | -CO- | - | 1 | N | Öl | |
| 36 | $H_2C=CH-$ | 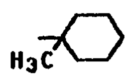 | -CHOH- | A | 1 | N | Öl | |
| 37 | $H_2C=CH-$ | 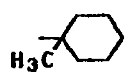 | -CO- | - | 2 | N | | |
| 38 | $H_2C=CH-$ | 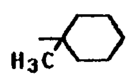 | -CHOH- | A | 2 | N | | |

| Beispiel Nr. | $R^1$ | $R^2$ | Z | Diastereom. | n | Y | Fp. (°C) | IR (Film) ($cm^{-1}$) |
|---|---|---|---|---|---|---|---|---|
| 39 | $H_2C=CH-$ | 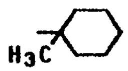 | -CHOH- | B | 2 | N | | |
| 40 | $H_2C=CCl-$ | 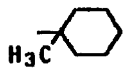 | -CO- | - | 1 | N | Öl | |
| 41 | $H_2C=CCl$ | 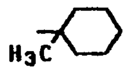 | -COOH- | A+B | 1 | N | Öl | |
| 42 | $H_3C-C(Cl)=CH-$ | 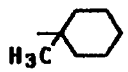 | -CO- | - | 1 | N | Öl | 2857, 1714, 1502, 1447, 1277, 1200, 1139, 1112, 1015, 678. |
| 43 | $H_3C-C(Cl)=CH-$ | 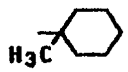 | -CHOH- | A+B | 1 | N | Öl | 3284, 2927, 2858, 1505, 1277, 1135, 1117, 1069, 679. |
| 44 | $H_3C-C(Cl)=CH-$ | 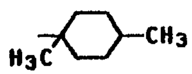 | -CO- | - | 1 | N | Öl | 2926, 2868, 1714, 1502, 1276, 1139, 1017, 678. |
| 45 | $H_3C-C(Cl)=CH-$ | 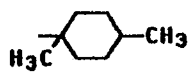 | -CHOH- | A+B | 1 | N | Öl | 3278, 2922, 2857, 1506, 1457, 1276, 1136, 679. |
| 46 | $CH_2=CH-$ | 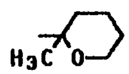 | -CH- | - | 1 | N | Öl | |
| 47 | $CH_2=CH-$ | 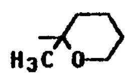 | -CHOH- | A+B | 1 | N | Öl | |
| 48 | $CH_2=CH-$ | 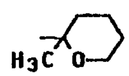 | -CO- | - | 2 | N | Öl | |

| Beispiel Nr. | $R^1$ | $R^2$ | Z | Diastereom. | n | Y | Fp. ($^0C$) | IR (Film) ($cm^{-1}$) |
|---|---|---|---|---|---|---|---|---|
| 49 | $CH_2=CH-$ | 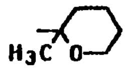 | -CHOH- | A | 2 | N | | |
| 50 | $CH_2=CH-$ | 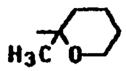 | -CHOH- | B | 2 | N | | |
| 51 | $H_2C=CCl-$ | 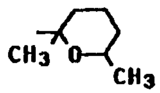 | -CO- | - | 1 | N | Öl | 2937, 1731, 1504, 1447, 1276, 1211, 1138, 1068, 1016, 678. |
| 52 | $H_2C=CCl-$ | 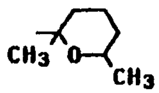 | -CHOH- | A | 1 | N | 83-85 | |
| 53 | $H_2C=CCl-$ | 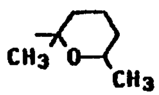 | -CHOH- | B | 1 | N | Öl | 3250, 2933, 1505, 1382, 1276, 1205, 1138, 1083, 1036, 895, 680. |
| 54 | $H_2C=CCl-$ | 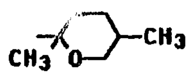 | -CO- | - | 1 | N | Öl | 2931, 1729, 1503, 1275, 1137, 1083, 1010, 953, 679. |
| 55 | $H_2C=CCl-$ | 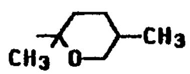 | -CHOH- | A+B | 1 | N | Öl | 3250, 2953, 2872, 1506, 1456, 1276, 1138, 1081, 1014, 679. |
| 56 | $H_2C=CCl-$ | tert.-$C_4H_9$ | 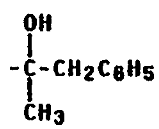 | A | 1 | N | 130-133 | |
| 57 | $H_2C=CCl-$ | tert.-$C_4H_9$ | 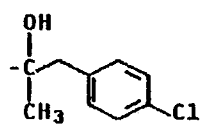 | A | 1 | N | 100-103 | |

| Beispiel Nr. | $R^1$ | $R^2$ | Z | Diastereom. | n | Y | Fp. ($^0C$) | IR (Film) ($cm^{-1}$) |
|---|---|---|---|---|---|---|---|---|
| 58 | Cl<br>$H_3C-C=CH-$ | tert.-$C_4H_9$ | 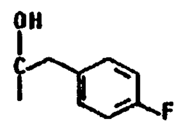 | A | 1 | N | 134-136 | |
| 59 | $CH_2=CH-$ | tert.-Butyl | C=O | - | 2 | N | Öl | 2972, 2936, 1718, 1502, 1369, 1276, 1140, 1008, 679. |
| 60 | $CH_2=CH-$ | tert.-Butyl | CHOH | A | 2 | N | 75-77 | |

| Beispiel Nr. | $R^1$ | $R^2$ | Z | Diastereom. | n | Y | Fp. (°C) | IR (Film) ($cm^{-1}$) oder Charaktr. $^1$H-NMR-Daten, $\sigma$ in ppm |
|---|---|---|---|---|---|---|---|---|
| 61 | $CH_2=CH-$ | tert.-Butyl | -CHOH- | B | 2 | N | 90-92 | |
| 62 | $CH_2=CBr-$ | tert.-Butyl | -C=O- | - | 2 | N | Öl | 2970, 2935, 1717, 1502, 1478, 1368, 1217, 1139, 1009, 678. |
| 63 | $CH_3CH=CH-$ | $CH_3C(=NOCH_3)-$ | -C=O- | - | 1 | N | Öl | 1,6 d (3), 1,95 s (3), 2,8 m (2), 4,1 d (3), 5,2-5,6 m (2), 7,95 s (1), 8,3 s (1). |
| 64 | $CH_3CH=CH-$ | $CH_3C(=NOCH_3)-$ | -CHOH- | AB-Gemisch | 1 | N | Öl | 1,55 d + 1,60 d (3), 1,8 s (3), 2,7 m (s), 3,8 s (3), 4,45 m (1), 5,1-5,6 m (2), 7,85 + 7,90 s (1), 8,1 + 8,15 s (1). |
| 65 | $CH_3CH=CH-$ | $CH_3C(=NOCH_3)-$ | -CHOH- | B | 1 | N | | |
| 66 | $CH_2=CH-$ | $CH_3C(=NOCH_3)-$ | -C=O- | - | 2 | N | Öl | 1,95 s (3), 4,1 s (3), 5,0 m (2), 5,75 m (1), 6,1 dd (1), 7,95 s (1), 8,35 s (1). |
| 67 | $CH_2=OH-$ | $CH_3C(=NOCH_3)-$ | -CHOH- | AB-Gemisch | 2 | N | Öl | 1,8 s (3), 1,8-2,3 m (4), 3,8 + 3,85 s (3), 4,55 m (1), 4,9-5,2 m (2), 5,6-5,85 m (1), 7,9-7,95 s (1), 8,2 s (1). |
| 68 | $CH_2=CH-$ | $CH_3C(=NOCH_3)-$ | -CHOH- | B | 2 | N | | |

| Beispiel Nr. | $R^1$ | $R^2$ | Z | Diastereom. | n | Y | Fp. (°C) | IR (Film) ($cm^{-1}$) oder Charaktr. $^1$-NMR-Daten, $\sigma$ in ppm |
|---|---|---|---|---|---|---|---|---|
| 69 | $CH_3CH=CH-$ | 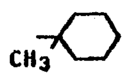 | -C=O- | - | 1 | N | Öl | 1,15 s (3), 1,6 d (3), 2,65 t (2), 5,2 m (1), 5,5 m (1), 5,55 t (1), 7,9 s (1), 8,3 s (1). |
| 70 | $CH_3CH=CH-$ | 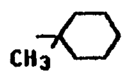 | -CHOH- | A | 1 | N | Öl | 0,5 s (3), 1,6 d (3), 2,65 m (2), 3,5 m (1), 4,55 m (1), 5,1-5,5 m (2), 7,9 s (1), 8,15 s (1). |
| 71 | $CH_3CH=CH-$ | 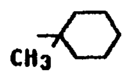 | -CHOH- | B | 1 | N | | |
| 72* | $CH_3CH=CH-$ | 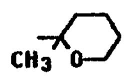 | -C=O- | - | 1 | N | Öl | 1,2 + 1,4 s (3), 1,55 d (3), 2,6-2,9 m (2), 5,2 m (1), 5,45 m (1), 5,8 + 5,9 m (1), 7,95 s (1), 8,25 - 8,30 s (1). |
| 73* | $CH_3CH=CH-$ | 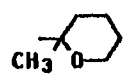 | -CHOH- | A | 1 | N | wachsart. | 0,8 + 0,82 s (1), 1,6 d (3), 2,55-2,85 m (2), 3,7 m (1), 4,65 m (1), 5,1-5,6 (2), 7,85-7,87 s (1), 8,25 + 8,27 s (1). |
| 74 | $CH_3CH=CH-$ | 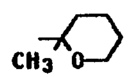 | -CHOH- | B | 1 | N | | |

*) Diastereomere

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse – wie Gurken, Bohnen und Kürbisgewächse –.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Hemileia vastatrix an Kaffee,
Alternaria solani an Kartoffeln, Tomaten,
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol) Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff. Die Aufwandmengen liegen je nach Art des gewünschten Effektes zwischen 0,02 und 3 kg Wirkstoff oder mehr je ha.

Die neuen Verbindungen können auch im Materialschutz eingesetzt werden. Bei der Anwendung der Wirkstoffe im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile lösemittelhaltiger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

III. 20 Gew.-Teile der Verbindung Nr. 7 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 13 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 14 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 21 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 23 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 25 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 43 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,

Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;

heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,

2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol,
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl-(5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol.

Für die folgenden Versuche wurde der bekannte Wirkstoff 2,2-Dimethyl-4-(1H-1,2,4-triazol-1-yl)-6-hepten-3-on (A) verwendet.

Versuch 1

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus besprüht. Nach etwa 20 Stunden werden die Versuchspflanzen mit wäßriger Spritzbrühe, die 80% Wirkstoff und 20% Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22°C und 70 bis 80% relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß bei der Anwendung als 0,025%ige Spritzbrühe beispielsweise die Verbindungen 7, 13, 14, 21, 23, 25, 43, 45 und 60 eine bessere fungizide Wirkung zeigen (97%) als der bekannte Wirkstoff A (50%).

Versuch 2

Wirksamkeit gegen Weizenbraunrost

Blätter von in Töpfen gewachsenen Weizensämlingen der Sorte "Jubilar" werden mit Sporen des Braunrostes (Puccinia recondita) bestäubt. Danach werden die Töpfe für 24 Stunden bei 20 bis 22°C in

eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit wäßrigen Spritzbrühen, die 80% Wirkstoff und 20% Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

Das Ergebnis des Versuches zeigt, daß bei der Anwendung als 0,025%ige Spritzbrühe beispielsweise die Verbindungen 23, 25, 43, 45 und 58 eine bessere fungizide Wirksamkeit zeigen (97%) als der bekannte Wirkstoff A (10%).

Versuch 3

Wirksamkeit gegen Botrytis cinerea an Paprika

Paprikasämlinge der Sorte "Neusiedler Ideal Elite" werden, nachdem sich 4–5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß bei der Anwendung als 0,05%ige Spritzbrühe beispielsweise die Verbindungen 11, 16, 43 und 70 eine bessere fungizide Wirkung zeigen (90%) als der bekannte Wirkstoff A (50%).

Die neuen Verbindungen der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation)
d) von den klimatischen Faktoren, z.B. Temperatur, Niederschlagsmenge und auch von der Tageslänge und der Lichtintensität,
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Decken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und – wegen der relativ geringen Blatt- bzw. Pflanzenmassen –, dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Blatt- und Sproßteil der Pflanze ist auch für ein gut kontrollierbares Entblättern von Nutzpflanzen wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird
- das Mikroklima im Pflanzenbestand durch einen kompakteren Wuchs günstig beeinflußt wird

Die Wirkstoffe können den Kulturpflanzen sowohl vom Samen her (als Saatgutbeizmittel) als auch über den Boden, d.h. durch die Wurzel sowie – besonders bevorzugt – durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,1 bis 12 kg/ha bevorzugt 0,25 bis 3 kg/ha als ausreichend zu betrachten.

Zur Bestimmung der wachstumsregulierenden Eigenschaft wurden Testpflanzen auf ausreichend mit Nährstoffen versorgtem Kultursubstrat in Kunststoffgefäßen (ca. 12,5 cm Durchmesser; Volumen ca. 500 ml) angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente CCC (US-3 156 554).

Gleichlaufend mit der Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen

Vergleichssubstanz:

CCC =

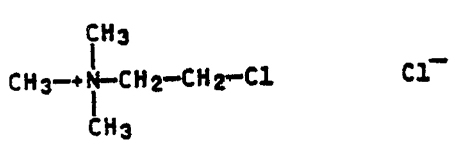

Tabelle 1: Sommergerste, Sorte "Aramir"
Vorauflauf-Bodenbehandlung

| Wirkstoff Nr. | mg Wirkstoff je Gefäß | Wuchshöhen relativ |
|---|---|---|
| unbehandelt | – | 100 |
| "A" | 6 | 87,7 |
| 1 | 6 | 69,3 |
| 6 | 6 | 75,3 |
| 9 | 6 | 60,2 |
| 10 | 6 | 52,6 |
| 12 | 6 | 60,2 |
| 43 | 6 | 80,9 |
| 59 | 6 | 71,8 |
| 60 | 6 | 64,6 |
| 61 | 6 | 57,4 |

Tabelle 2: Rasen
Nachauflauf-Blattbehandlung

| Wirkstoff Nr. | mg Wirkstoff je Gefäß | Wuchshöhen relativ |
|---|---|---|
| unbehandelt | – | 100 |
| "A" | 1,5 | 90,3 |
| | 6 | 87,1 |
| 6 | 1,5 | 80,7 |
| | 6 | 61,3 |

Tabelle 3: Sonnenblumen "Sorex"
Nachauflauf-Blattbehandlung

| Wirkstoff Nr. | mg Wirkstoff je Gefäß | Wuchshöhen relativ |
|---|---|---|
| unbehandelt | – | 100 |
| "A" | 1,5 | 97,6 |
| | 6 | 87,2 |
| 43 | 1,5 | 92,5 |
| | 6 | 75,4 |
| 45 | 1,5 | 89,1 |
| | 6 | 82,2 |
| 51 | 1,5 | 90,1 |
| | 6 | 75,1 |
| 54 | 1,5 | 82,6 |
| | 6 | 75,1 |

Tabelle 4: Sommerraps "Petranova"
Vorauflauf-Bodenbehandlung

| Wirkstoff Nr. | mg Wirkstoff je Gefäß | Wuchshöhen relativ |
|---|---|---|
| unbehandelt | – | 100 |
| "A" | 6 | 92,5 |
| 6 | 6 | 84,8 |
| 7 | 6 | 71,4 |
| 9 | 6 | 76,2 |
| 10 | 6 | 52,4 |
| 12 | 6 | 81,0 |
| 13 | 6 | 81,8 |
| 16 | 6 | 63,6 |
| 17 | 6 | 81,8 |
| 42 | 6 | 85,6 |
| 45 | 6 | 77,3 |
| 60 | 6 | 81,8 |
| 61 | 6 | 81,8 |

Tabelle 5: Sommerraps "Petranova"
Nachauflauf-Blattbehandlung

| Wirkstoff Nr. | mg Wirkstoff je Gefäß | Wuchshöhen relativ |
|---|---|---|
| unbehandelt | – | 100 |
| "A" | 1,5 | 97,5 |
| | 6 | 89,4 |
| 42 | 1,5 | 92,4 |
| | 6 | 68,7 |
| 44 | 1,5 | 82,9 |
| | 6 | 73,4 |
| 45 | 1,5 | 92,4 |
| | 6 | 75,8 |
| 56 | 1,5 | 77,4 |
| | 6 | 64,5 |
| 57 | 1,5 | 73,1 |
| | 6 | 55,9 |
| 58 | 1,5 | 86,0 |
| | 6 | 60,2 |

Tabelle 6

Reiskeimlingtest

In weiteren Untersuchungen wurden junge Reiskeimlinge (Sorte Girona) in einer Nährlösung herangezogen, die die jeweiligen Wirkstoffe in unterschiedlichen Konzentrationen enthielt. Nach sechs Tagen Wachstum bei 25°C im Dauerlicht wurde die Wirkstoffkonzentration bestimmt, die das Längenwachstum der zweiten Blattscheide um 50% vermindert (= $KI_{50}$).
(Details bei: W. Rademacher und J. Jung, Berichte aus dem Fachgebiet Herbologie, Heft 24, pp 127–134, Universität Hohenheim, 1983).

| Wirkstoff Nr. | $KI_{50}$ (molar) |
|---|---|
| "A" | $1,5 \times 10^{-2}$ |
| 10 | $1,2 \times 10^{-4}$ |
| 13 | $7,9 \times 10^{-5}$ |
| 14 | $1,4 \times 10^{-4}$ |
| 51 | $5,0 \times 10^{-5}$ |
| 57 | $2,4 \times 10^{-5}$ |
| 58 | $2,8 \times 10^{-4}$ |

**Patentansprüche**

1. Azolverbindungen der Formel I

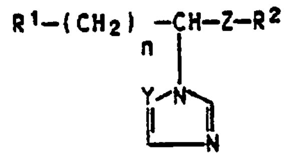

I,

worin
$R^1$ eine Gruppe der Formel

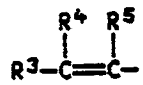

mit cis- oder trans-Doppelbindung bedeutet, wobei
$R^3$, $R^4$ und $R^5$ gleich oder verschieden sind und Wasserstoff, Fluor, Chlor, Brom, Methyl oder Ethyl bedeuten,
n eine ganze Zahl von 1 bis 3 bedeutet,
Y $\geq$CH oder $\geq$N ist,
$R^2$ tert.-Butyl, 5-Methyl-1,3-dioxan-5-yl, ggf. durch Methyl substituiertes 1-Methylcyclohexyl und ggf. durch Methyl substituiertes 2-Methyltetrahydropyran-2-yl oder die Gruppe

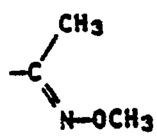

bedeutet, und
Z die Gruppe >C=O, >C=N–$OR^8$ oder >$CR^6$–$OR^7$ bedeutet, mit der Maßgabe, daß nicht gleichzeitig $R^1$ $CH_2$=CH–, n = 1, Z >C=O und $R^2$ tert.-Butyl ist, und
$R^6$ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
$R^7$ Wasserstoff, $C_2$–$C_4$-Alkanoyl oder Benzoyl, $R^8$ Wasserstoff oder Methyl bedeutet, oder deren für Pflanzen verträglichen Säureadditionssalze oder Metall-Komplexe.

2. Verfahren zur Herstellung von Azolverbindungen der Formel I gemäß Anspruch 1, worin Z >$CR^6$–$OR^7$ bedeutet und $R^6$ Wasserstoff ist, dadurch gekennzeichnet, daß man ein Keton der Formel II

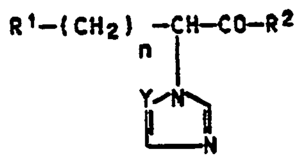

II,

vorzugsweise stereoselektiv reduziert und den erhaltenen sekundären Alkohol gegebenenfalls mit einem $C_2$–$C_4$-Alkanoylhalogenid oder -anhydrid umsetzt.

3. Verfahren zur Herstellung von Azolverbindungen der Formel I gemäß Anspruch 1 bzw. 2, worin Z >$CR^6$–$OR^7$ bedeutet und $R^6$ Methyl, Vinyl, Allyl oder Benzyl ist, dadurch gekennzeichnet, daß man ein Keton der Formel II gemäß Anspruch 2 mit einer den Substituenten $R^6$ einführenden Grignard- oder Lithiumverbindung umsetzt, das erhaltene Alkoholat hydrolysiert und den erhaltenen tertiären Alkohol gegebenenfalls mit einem $C_2$–$C_4$-Alkanoylhalogenid oder -anhydrid umsetzt.

4. Verfahren zur Bekämpfung von Pilzen, dadurch gekennzeichnet, daß man die Pilze oder die von Pilzbefall bedrohten Pflanzen, Materialien, Saatgüter oder den Boden mit einer fungizid wirksamen Menge einer Azolverbindung der Formel I behandelt

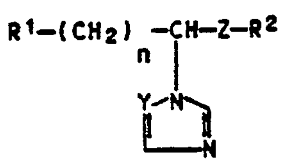

I,

worin
$R^1$ eine Gruppe der Formel

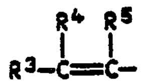

mit cis- oder trans-Doppelbindung bedeutet, wobei
$R^3$, $R^4$ und $R^5$ gleich oder verschieden sind und Wasserstoff, Fluor, Chlor, Brom, Methyl oder Ethyl bedeuten,
n eine ganze Zahl von 1 bis 3 bedeutet,
Y ≥CH oder ≥N ist,
$R^2$ tert.-Butyl, 5-Methyl-1,3-dioxan-5-yl, ggf. durch Methyl substituiertes 1-Methylcyclohexyl und ggf. durch Methyl substituiertes 2-Methyltetrahydropyran-2-yl oder die Gruppe

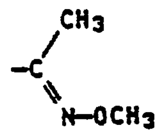

bedeutet, und
Z die Gruppe >C=O, >C=N–$OR^8$ oder >$CR^6$–$OR^7$ bedeutet, mit der Maßgabe, daß nicht gleichzeitig $R^1$ $CH_2$=CH–, n = 1, Z >C=O und $R^2$ tert.-Butyl ist, und
$R^6$ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
$R^7$ Wasserstoff, $C_2$–$C_4$-Alkanoyl oder Benzoyl, $R^8$ Wasserstoff oder Methyl bedeutet, oder deren für Pflanzen verträglichen Säureadditionssalze oder Metall-Komplexe.

5. Verfahren zur Regulierung des Pflanzenwachstums, dadurch gekennzeichnet, daß man eine Verbindung der Formel I

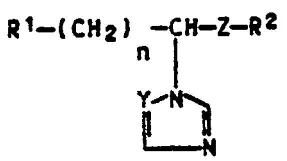

I,

worin
$R^1$ eine Gruppe der Formel

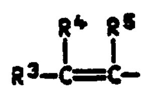

mit cis- oder trans-Doppelbindung bedeutet, wobei
$R^3$, $R^4$ und $R^5$ gleich oder verschieden sind und Wasserstoff, Fluor, Chlor, Brom, Methyl oder Ethyl bedeuten,
n eine ganze Zahl von 1 bis 3 bedeutet,
Y ≥CH oder ≥N ist,
$R^2$ tert.-Butyl, 5-Methyl-1,3-dioxan-5-yl, ggf. durch Methyl substituiertes 1-Methylcyclohexyl und ggf. durch Methyl substituiertes 2-Methyltetrahydropyran-2-yl oder die Gruppe

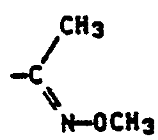

bedeutet, und
Z die Gruppe >C=O, >C=N–$OR^8$ oder >$CR^6$–$OR^7$ bedeutet, mit der Maßgabe, daß nicht gleichzeitig $R^1$ $CH_2$=CH–, n = 1, Z >C=O und $R^2$ tert.-Butyl ist, und
$R^6$ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
$R^7$ Wasserstoff, $C_2$–$C_4$-Alkanoyl oder Benzoyl, $R^8$ Wasserstoff oder Methyl bedeutet, oder deren für Pflanzen verträglichen Säureadditionssalze oder Metall-Komplexe auf Pflanzen, deren Lebensraum oder auf Saatgut einwirken läßt.

6. Fungizides Mittel enthaltend einen Trägerstoff und eine fungizid wirksame Menge einer Azolverbindung der Formel I

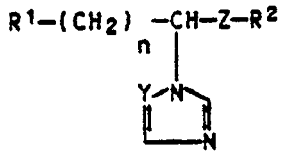

I,

worin
$R^1$ eine Gruppe der Formel

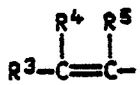

mit cis- oder trans-Doppelbindung bedeutet, wobei
$R^3$, $R^4$ und $R^5$ gleich oder verschieden sind und Wasserstoff, Fluor, Chlor, Brom, Methyl oder Ethyl bedeuten,
n eine ganze Zahl von 1 bis 3 bedeutet,
Y ≥CH oder ≥N ist,
$R^2$ tert.-Butyl, 5-Methyl-1,3-dioxan-5-yl, ggf. durch Methyl substituiertes 1-Methylcyclohexyl und ggf. durch Methyl substituiertes 2-Methyltetrahydropyran-2-yl oder die Gruppe

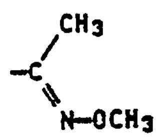

bedeutet, und
Z die Gruppe >C=O, >C=N–$OR^8$ oder >$CR^6$–$OR^7$ bedeutet, mit der Maßgabe, daß nicht gleichzeitig $R^1$ $CH_2$=CH–, n = 1, Z >C=O und $R^2$ tert.-Butyl ist, und
$R^6$ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
$R^7$ Wasserstoff, $C_2$–$C_4$-Alkanoyl oder Benzoyl, $R^8$ Wasserstoff oder Methyl bedeutet, oder deren für Pflanzen verträglichen Säureadditionssalze oder Metall-Komplexe.

7. Mittel zur Regulierung des Pflanzenwachstums, enthaltend einen Trägerstoff und eine Azolverbindung der Formel I

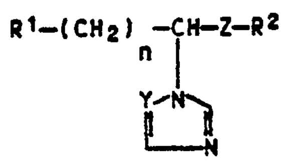

I,

worin
$R^1$ eine Gruppe der Formel

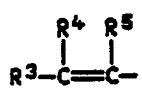

mit cis- oder trans-Doppelbindung bedeutet, wobei
$R^3$, $R^4$ und $R^5$ gleich oder verschieden sind und Wasserstoff, Fluor, Chlor, Brom, Methyl oder Ethyl bedeuten,
n eine ganze Zahl von 1 bis 3 bedeutet,
Y ≥CH oder ≥N ist,
$R^2$ tert.-Butyl, 5-Methyl-1,3-dioxan-5-yl, ggf. durch Methyl substituiertes 1-Methylcyclohexyl und ggf. durch Methyl substituiertes 2-Methyltetrahydropyran-2-yl oder die Gruppe

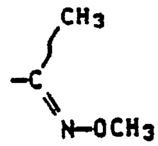

bedeutet, und
Z die Gruppe >C=O, >C=N–OR⁸ oder >CR⁶–OR⁷ bedeutet, mit der Maßgabe, daß nicht gleichzeitig $R^1$ $CH_2$=CH–, n = 1, Z >C=O und $R^2$ tert.-Butyl ist, und
$R^6$ Wasserstoff, Methyl, Vinyl, Allyl oder Benzyl bedeutet, wobei der Benzylrest gegebenenfalls durch Fluor, Chlor, Brom, Trifluormethyl, Methyl, Ethyl, Propyl, Butyl oder Methoxy substituiert sein kann, und
$R^7$ Wasserstoff, $C_2$–$C_4$-Alkanoyl oder Benzoyl, $R^8$ Wasserstoff oder Methyl bedeutet, oder deren für Pflanzen verträglichen Säureadditionssalze oder Metall-Komplexe.

8. Azolverbindung gemäß Anspruch 1, dadurch gekennzeichnet, daß
$R^1$ Ethenyl, Chlorethenyl, Bromethenyl, Propenyl, Chlorpropenyl,
$R^2$ tertiär-Butyl, 5-Methyl-1,3-dioxan-5-yl, 1-Methylcyclohexyl,
n die Zahl 1,
Y Stickstoff und
Z die Gruppe CO oder CHOH bedeutet.

## Claims

1. An azole compound of the formula I

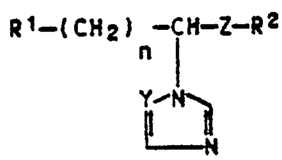

I,

where
$R^1$ is a group of the formula

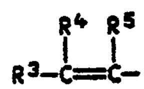

having a cis or trans double bond,
$R^3$, $R^4$ and $R^5$ are identical or different and are each hydrogen, fluorine, chlorine, bromine, methyl or ethyl,
n is an integer from 1 to 3,
Y is ≥CH or ≥N,
$R^2$ is tert-butyl, 5-methyl-1,3-dioxan-5-yl, unsubstituted or methyl-substituted 1-methylcyclohexyl, unsubstituted or methyl-substituted 2-methyltetrahydropyran-2-yl or the group

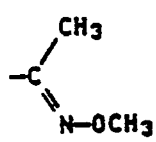

Z is the group >C=O, >C=N–OR⁸ or >CR⁶–OR⁷, with the exception of compounds in which $R^1$ is $CH_2$=CH–, n is 1, Z is >C=O and $R^2$ is tert-butyl,
$R^6$ is hydrogen, methyl, vinyl, allyl or benzyl, where the benzyl radical may be substituted by fluorine, chlorine, bromine, trifluoromethyl, methyl, ethyl, propyl, butyl or methoxy,
$R^7$ is hydrogen, $C_2$–$C_4$-alkanoyl or benzoyl, $R^8$ is hydrogen or methyl, or a plant-tolerated acid addition salt or metal complex thereof.

2. A process for the preparation of an azole compound of the formula I as claimed in claim 1, where Z is >CR⁶–OR⁷ and $R^6$ is hydrogen, wherein a ketone of the formula II

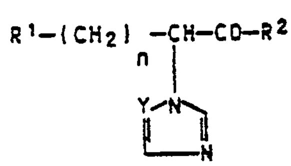

II,

is reduced, preferably stereoselectively, and the resulting secondary alcohol is, if required, reacted with a $C_2$–$C_4$-alkanoyl halide or anhydride.

3. A process for the preparation of an azole compound of the formula I as claimed in claim 1 or 2, where Z is >$CR^6$–$OR^7$ and $R^6$ is methyl, vinyl, allyl or benzyl, wherein a ketone of the formula II as claimed in claim 2 is reacted with a Grignard or lithium compound which introduces the substituent $R^6$, the resulting alcoholate is hydrolyzed and the tertiary alcohol obtained is, if required, reacted with a $C_2$–$C_4$-alkanoyl halide or anhydride.

4. A method for controlling fungi, which comprises treating the fungi or the plants, materials or seeds threatened by fungal attack, or the soil, with a fungicidally effective amount of an azole compound of the formula I

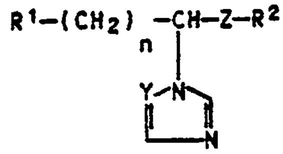

I,

where

$R^1$ is a group of the formula

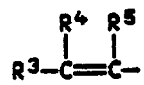

having a cis or trans double bond,

$R^3$, $R^4$ and $R^5$ are identical or different and are each hydrogen, fluorine, chlorine, bromine, methyl or ethyl,

n is an integer from 1 to 3,

Y is ≥CH or ≥N,

$R^2$ is tert-butyl, 5-methyl-1,3-dioxan-5-yl, unsubstituted or methyl-substituted 1-methylcyclohexyl, unsubstituted or methyl-substituted 2-methyltetrahydropyran-2-yl or the group

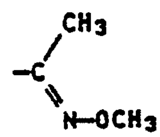

Z is the group >C=O, >C=N–$OR^8$ or >$CR^6$–$OR^7$, with the exception of compounds in which $R^1$ is $CH_2$=CH–, n is 1, Z is C=O and $R^2$ is tert-butyl,

$R^6$ is hydrogen, methyl, vinyl, allyl or benzyl, where the benzyl radical may be substituted by fluorine, chlorine, bromine, trifluoromethyl, methyl, ethyl, propyl, butyl or methoxy,

$R^7$ is hydrogen, $C_2$–$C_4$-alkanoyl or benzoyl, $R^8$ is hydrogen or methyl, or a plant-tolerated acid addition salt or metal complex thereof.

5. A method for regulating plant growth, wherein a compound of the formula I

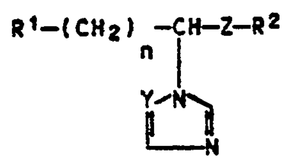

I,

where

$R^1$ is a group of the formula

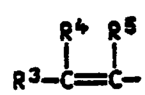

having a cis or trans double bond,

$R^3$, $R^4$ and $R^5$ are identical or different and are each hydrogen, fluorine, chlorine, bromine, methyl or ethyl,

n is an integer from 1 to 3,

Y is ≥CH or ≥N,

$R^2$ is tert-butyl, 5-methyl-1,3-dioxan-5-yl, unsubstituted or methyl-substituted 1-methylcyclohexyl, unsubstituted or methyl-substituted 2-methyltetrahydropyran-2-yl or the group

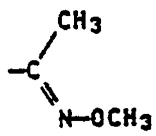

Z is the group >C=O, >C=N–OR⁸ or >CR⁶–OR⁷, with the exception of compounds in which $R^1$ is $CH_2$=CH–, n is 1, Z is >C=O and $R^2$ is tert-butyl,
$R^6$ is hydrogen, methyl, vinyl, allyl or benzyl, where the benzyl radical may be substituted by fluorine, chlorine, bromine, trifluoromethyl, methyl, ethyl, propyl, butyl or methoxy,
$R^7$ is hydrogen, $C_2$–$C_4$-alkanoyl or benzoyl, $R^8$ is hydrogen or methyl, or a plant-tolerated acid addition salt or metal complex thereof, is allowed to act on plants or their habitat or on seed.

6. A fungicide containing a carrier and a fungicidally effective amount of an azole compound of the formula I

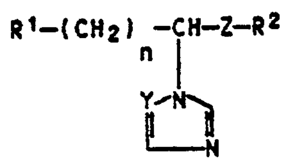

I,

where
$R^1$ is a group of the formula

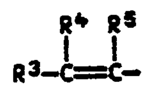

having a cis or trans double bond,
$R^3$, $R^4$ and $R^5$ are identical or different and are each hydrogen, fluorine, chlorine, bromine, methyl or ethyl,
n is an integer from 1 to 3,
Y is ≥CH or ≥N,
$R^2$ is tert-butyl, 5-methyl-1,3-dioxan-5-yl, unsubstituted or methyl-substituted 1-methylcyclohexyl, unsubstituted or methyl-substituted 2-methyltetrahydropyran-2-yl or the group

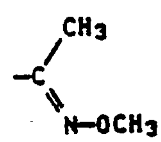

Z is the group >C=O, >C=N–OR⁸ or >CR⁶–OR⁷, with the exception of compounds in which $R^1$ is $CH_2$=CH–, n is 1, Z is >C=O and $R^2$ is tert-butyl,
$R^6$ is hydrogen, methyl, vinyl, allyl or benzyl, where the benzyl radical may be substituted by fluorine, chlorine, bromine, trifluoromethyl, methyl, ethyl, propyl, butyl or methoxy,
$R^7$ is hydrogen, $C_2$–$C_4$-alkanoyl or benzoyl, $R^8$ is hydrogen or methyl, or a plant-tolerated acid addition salt or metal complex thereof.

7. A plant growth regulator, containing a carrier and an azole compound of the formula I

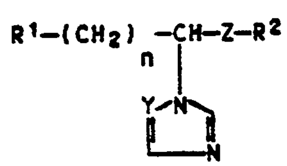

I,

where
$R^1$ is a group of the formula

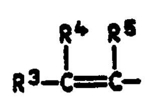

having a cis or trans double bond,
$R^3$, $R^4$ and $R^5$ are identical or different and are each hydrogen, fluorine, chlorine, bromine, methyl or ethyl,
n is an integer from 1 to 3,

Y is ≥CH or ≥N,
$R^2$ is tert-butyl, 5-methyl-1,3-dioxan-5-yl, unsubstituted or methyl-substituted 1-methylcyclohexyl, unsubstituted or methyl-substituted 2-methyltetrahydropyran-2-yl or the group

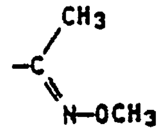

Z is the group >C=O, >C=N–$OR^8$ or >$CR^6$–$OR^7$, with the exception of compounds in which $R^1$ is $CH_2$=CH–, n is 1, Z is >C=O and $R^2$ is tert-butyl,
$R^6$ is hydrogen, methyl, vinyl, allyl or benzyl, where the benzyl radical may be substituted by fluorine, chlorine, bromine, trifluoromethyl, methyl, ethyl, propyl, butyl or methoxy,
$R^7$ is hydrogen, $C_2$–$C_4$-alkanoyl or benzoyl, $R^8$ is hydrogen or methyl, or a plant-tolerated acid addition salt or metal complex thereof.

8. An azole compound as claimed in claim 1, wherein
$R^1$ is ethenyl, chloroethenyl, bromoethenyl, propenyl, or chloropropenyl,
$R^2$ is tert-butyl, 5-methyl-1,3-dioxan-5-yl or 1-methylcyclohexyl,
n is 1,
Y is nitrogen and
Z is CO or CHOH.

**Revendications**

1. Composés azoles de la formule I

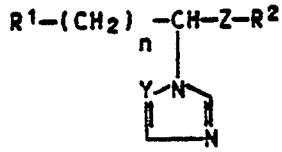

I,

dans laquelle
$R^1$ représente un groupe de formule

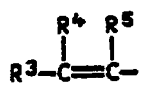

a double liaison cis ou trans,
$R^3$, $R^4$ et $R^5$ étant identiques ou différents et représentant hydrogène, fluor, chlore, brome, méthyle ou éthyle
n est un nombre entier de 1 à 3
Y est ≥CH ou ≥N
$R^2$ représente tert-butyle, 5-méthyl-1,3-dioxane-5-yle, 1-méthylcyclohéxyle étant substitué par méthyle et 2-méthyltétrahydropyrane-2-yle éventuellement substitué par méthyle, ou le groupe

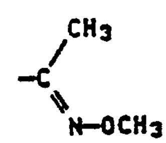

et
Z représente le groupe >C=O, >C=N–$OR^8$ ou >$CR^6$–$OR^7$ sous réserve qu'il n'y ait pas simultanément $R^1$, $CH_2$=CH–, n = 1, Z >C=O et $R^2$, tert.-butyle et
$R^6$ représente hydrogène, méthyle, vinyle, allyle ou benzyle, le reste benzyle pouvant être substitué éventuellement par fluor, chlore, brome, trifluorométhyle, méthyle, éthyle, propyle, butyle ou méthoxy, et
$R^7$ représente hydrogène, alcanoyle en $C_2$–$C_4$ ou benzoyle, $R^8$, hydrogène ou méthyle, ou leurs sels d'addition d'acides ou complexes métalliques compatibles avec les plantes.

2. Procédé de préparation de composés azoles de formule I selon la revendication 1, dans laquelle Z représente >$CR^6$–$OR^7$ et $R^6$ est l'hydrogène, caractérisé par le fait qu'on réduit de préférence stéréosélectivement une cétone de formule II

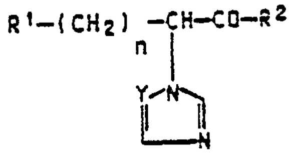

II.

et l'on fait réagir l'alcool secondaire obtenu, éventuellement avec un halogénure ou anhydride d'alcanoyle en $C_2$–$C_4$.

3. Procédé de préparation de composés azolés de formule I selon la revendication 1 ou 2, où Z représente >$CR^6$–$OR^7$ et $R^6$ est méthyle, vinyle, allyle ou benzyle, caractérisé par le fait que l'on fait réagir une cétone de formule II selon la revendication 2 avec un composé de lithium ou de grignard introduisant le substituant $R^6$, ou hydrolyse l'alcoolat obtenu, et on fait réagir l'alcool tertiaire obtenu, éventuellement avec un halogénure ou anhydride d'alcanoyle en $C_2$–$C_4$.

4. Procédé de lutte contre les champignons, caractérisé par le fait que l'on traite les champignons ou les plantes, matériaux, semences ou le sol, menacés par l'attaque des champignons avec une quantité efficace du point de vue fongicide, d'un composé azole de la formule I

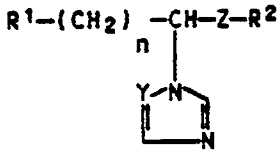

I,

dans laquelle
$R^1$ représente un groupe de formule

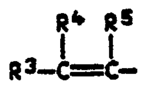

à double liaison cis ou trans,
$R^3$, $R^4$ et $R^5$ étant identiques ou différents et représentant hydrogène, fluor, chlore, brome, méthyle ou éthyle
n est un nombre entier de 1 à 3
Y est ≥CH ou ≥N
$R^2$ représente tert-butyle, 5-méthyl-1,3-dioxane-5-yle, 1-méthylcyclohéxyle étant substitué par méthyle et 2-méthyltétrahydropyrane-2-yle éventuellement substitué par méthyle, ou le groupe

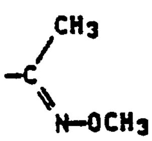

et
Z représente le groupe >C=O, >C=N–$OR^8$ ou >$CR^6$–$OR^7$ sous réserve qu'il n'y ait pas simultanément $R^1$, $CH_2$=CH–, n = 1, Z >C=O et $R^2$, tert-butyle et
$R^6$ représente hydrogène, méthyle, vinyle, allyle ou benzyle, le reste benzyle pouvant être substitué éventuellement par fluor, chlore, brome, trifluorométhyle, méthyle, éthyle, propyle, butyle ou méthoxy, et
$R^7$ représente hydrogène, alcanoyle en $C_2$–$C_4$ ou benzoyle, $R^8$, hydrogène ou méthyle, ou leurs sels d'addition d'acides ou complexes métalliques compatibles avec les plantes.

5. Procédé de régulation de la croissance des plantes, caractérisé par le fait que l'on fait agir sur les plantes, leur biotope ou les semences, un composé azole de la formule I

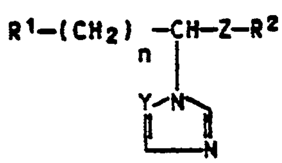

I,

dans laquelle
$R^1$ représente un groupe de formule

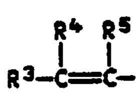

à double liaison cis ou trans,
$R^3$, $R^4$ et $R^5$ étant identiques ou différents et représentant hydrogène, fluor, chlore, brome, méthyle ou éthyle
n est un nombre entier de 1 à 3
Y est ≥CH ou ≥N
$R^2$ représente tert-butyle, 5-méthyl-1,3-dioxane-5-yle, 1-méthylcyclohéxyle étant substitué par méthyle et 2-méthyltétrahydropyrane-2-yle éventuellement substitué par méthyle, ou le groupe

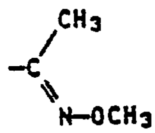

et
Z représente le groupe >C=O, >C=N–$OR^8$ ou >$CR^6$–$OR^7$ sous réserve qu'il n'y ait pas simultanément $R^1$, $CH_2$=CH–, n = 1, Z >C=O et $R^2$, tert.-butyle et
$R^6$ représente hydrogène, méthyle, vinyle, allyle ou benzyle, le reste benzyle pouvant être substitué éventuellement par fluor, chlore, brome, trifluorométhyle, méthyle, éthyle, propyle, butyle ou méthoxy, et
$R^7$ représente hydrogène, alcanoyle en $C_2$–$C_4$ ou benzoyle, $R^8$, hydrogène ou méthyle, ou leurs sels d'addition d'acides ou complexes métalliques compatibles avec les plantes.

6. Agent fongicide contenant un support et une quantité efficace du point de vue fongicide d'un composé azole de la formule I

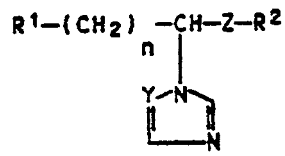

I,

dans laquelle
$R^1$ représente un groupe de formule

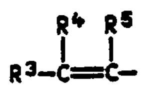

à double liaison cis ou trans,
$R^3$, $R^4$ et $R^5$ étant identiques ou différents et représentant hydrogène, fluor, chlore, brome, méthyle ou éthyle
n est un nombre entier de 1 à 3
Y est ≥CH ou ≥N
$R^2$ représente tert-butyle, 5-méthyl-1,3-dioxane-5-yle, 1-méthylcyclohéxyle étant substitué par méthyle et 2-méthyltétrahydropyrane-2-yle éventuellement substitué par méthyle, ou le groupe

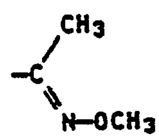

et
Z représente le groupe >C=O, >C=N–$OR^8$ ou >$CR^6$–$OR^7$ sous réserve qu'il n'y ait pas simultanément $R^1$, $CH_2$=CH–, n = 1, Z >C=O et $R^2$, tert.-butyle et
$R^6$ représente hydrogène, méthyle, vinyle, allyle ou benzyle, le reste benzyle pouvant être substitué éventuellement par fluor, chlore, brome, trifluorométhyle, méthyle, éthyle, propyle, butyle ou méthoxy, et
$R^7$ représente hydrogène, alcanoyle en $C_2$–$C_4$ ou benzoyle, $R^8$, hydrogène ou méthyle, ou leurs sels d'addition d'acides ou complexes métalliques compatibles avec les plantes.

7. Agent de régulation de la croissance des plantes contenant un support et une quantité efficace du point de vue fongicide d'un composé azole de la formule I

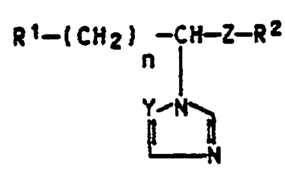

I,

dans laquelle

$R^1$ représente un groupe de formule

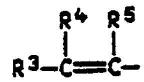

à double liaison cis ou trans,
$R^3$, $R^4$ et $R^5$ étant identiques ou différents et représentant hydrogène, fluor, chlore, brome, méthyle ou éthyle
n est un nombre entier de 1 à 3
Y est ≥CH ou ≥N
$R^2$ représente tert-butyle, 5-méthyl-1,3-dioxane-5-yle, 1-méthylcyclohéxyle étant substitué par méthyle et 2-méthyltétrahydropyrane-2-yle éventuellement substitué par méthyle, ou le groupe

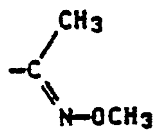

et
Z représente le groupe >C=O, >C=N–$OR^8$ ou >$CR^6$–$OR^7$ sous réserve qu'il n'y ait pas simultanément $R^1$, $CH_2$=CH–, n = 1, Z >C=O et $R^2$, tert-butyle et
$R^6$ représente hydrogène, méthyle, vinyle, allyle ou benzyle, le reste benzyle pouvant être substitué éventuellement par fluor, chlore, brome, trifluorométhyle, méthyle, éthyle, propyle, butyle ou méthoxy, et
$R^7$ représente hydrogène, alcanoyle en $C_2$–$C_4$ ou benzoyle, $R^8$, hydrogène ou méthyle, ou leurs sels d'addition d'acides ou complexes métalliques compatibles avec les plantes.

8. Composés azole selon la revendication 1, caractérisé par le fait que
$R^1$ représente éthényle, chloroéthényle, brométhényle, propényle, chloropropényle
$R^2$ tert.-butyle, 5-méthyle-1,3-dioxan-5-yle, 1-méthylcyclohéxyle
n le nombre 1
Y azote et
Z le groupe CO ou CHOH.